# EUROPEAN PATENT SPECIFICATION

(11) **EP 2 837 347 B1**
(45) Date of publication and mention of the grant of the patent: **03.10.2018**
(21) Application number: 14192707.9
(22) Date of filing: 30.01.2012
(51) Int. Cl.: A61B 17/70

(54) **Bone anchoring device**
Knochenverankerungsvorrichtung
Dispositif d'ancrage d'os

(43) Date of publication of application: 18.02.2015
(62) Divisional of application: 12153154.5
(73) Proprietor: Biedermann Technologies GmbH & Co. KG, 78166 Donaueschingen (DE)
(72) Inventor: Matthis, Wilfried, 79367 Weisweil (DE); Meer, Martin, 72189 Vöhringen (DE); Biedermann, Lutz, 78048 VS-Villingen (DE)
(74) Representative: Prüfer & Partner mbB Patentanwälte · Rechtsanwälte

(56) References cited:
- EP-A1- 2 371 311
- EP-A1- 2 455 028
- EP-A1- 2 468 198
- WO-A1-2012/064360
- US-A1- 2010 191 293
- US-A1- 2010 234 902
- US-B2- 7 604 656

## Description

The invention relates to a bone anchoring device comprising a bone anchoring element having a head and a shaft for anchoring in the bone, a receiving part for coupling the bone anchoring element to a rod, the receiving part comprising an accommodation space for accommodating the head and a bore being in communication with the accommodation space, the bore having a bore axis, a pressure member configured to move in the bore and comprising a first surface for engaging the head and a second surface on which the rod acts, wherein the pressure member is configured to assume a first position in which it exerts a preload onto the head that results from friction between the first surface and the head to enable the shaft to be maintained in a desired angular position before locking the head in the receiving part, and a second position in which the head is locked with respect to the receiving part, and wherein the first position is obtained by moving the pressure member by the action of a predefined force acting onto the pressure member in an axial direction and wherein the pressure member is maintained in the first position by interaction with the receiving part and can be released from the first position through action of an axial force.

US 2007/0118123 A1 describes a polyaxial bone anchor with increased angulation. The 20 polyaxial bone anchor has a locking element shaped and configured to allow an anchoring member e.g. a screw or hook to polyaxially rotate at large angles about a central axis of the bone anchor before compression locking the anchoring member within an anchor head.

US 7,604,656 B2 describes an apparatus comprising a fastener, a housing having a passage, and a spacer received in the passage and engageable with the fastener, wherein pin members retain the spacer and the fastener in the housing and wherein an end portion of the pin members has a tapered surface by which the spacer is urged axially toward the fastener when the pin member is inserted through the housing. The pin members also apply an axial force to the spacer to prevent relative movement between the spacer and the housing when the rod is disengaged from the spacer and the spacer engages the fastener. The pin members hold the spacer in frictional engagement with the fastener.

Although the polyaxial bone anchoring device described above ensures an enlarged angulation in a desired orientation, there is still a need for an improved polyaxial bone anchoring device in terms of simplicity of the design and handling of the device.

EP 2 371 311 A1 describes a bone anchoring device according to the preamble of claim 1 and shows a receiving part with a bore and projections formed therein which respectively frictionally engage notches of a pressure element inserted into the bore, wherein a preload is exerted onto the spherical head of anchoring element. The projections are formed by crimping.

US 2010/191293 A1 describes a medical implant assembly that includes a polyaxial bone anchor having a shank with an upper portion, a receiver, a retainer for holding the shank upper portion in the receiver, a lower compression insert with surfaces for closely receiving an elongate connecting member and a closure structure that may independently engage the lower compression insert to lock the shank with respect to the receiver while selectively not locking the elongate member. The walls of the receiver may be crimped at a location with respect to the lower compression insert that causes the insert to bias against and frictionally engage a domed a surface of the shank to provide a sub-assembly in which the shank is pivotable with respect to the receiver, but in a non-floppy manner at the desired articulation with respect to the shank and have the assembly hold such desired position prior to insertion of the rod.

It is the object of the invention to provide an improved polyaxial bone anchoring device.

The object is solved by a polyaxial bone anchoring device according to claim 1 and by a method for assembling a polyaxial bone anchoring device according to claim 7. Further developments are given in the dependent claims.

With the polyaxial bone anchoring device a temporary clamping of the head with an exactly predetermined force in a desired angular position with respect to the receiving part without locking the head can be achieved. In this condition, the pressure member exerts a preload force onto the head in which the head is not locked but prevented from freely pivoting by friction. The preload is achieved by applying an axial force on the pressure member. The preload is then maintained by a radial force which acts on the pressure member and frictionally holds the pressure member in position with respect to the receiving part. When the head is temporarily clamped, the alignment of the receiving part with respect to the rod and the insertion of the rod is facilitated, in particular in a situation in which a multitude of bone anchors have to be connected to the rod.

The mechanism to frictionally maintain the position of the head before locking is free from any spring members or portions. The polyaxial bone anchoring device has few parts, which are of simple design. According to an embodiment, for achieving the preload onto the head no further parts are required due to the interference fit connection. Referring to the interference fit connection the radial forces, i.e. in a 90° angle to the longitudinal axis of the receiving part, result from the elastic deformation of the material. The bone anchoring device can be manufactured easily and cost-effectively to. Furthermore, existing receiving parts can be used without having to redesign their shape. Only the pressure members have to be adapted in that an interference fit between an outer diameter of the pressure member and an inner diameter of the receiving part is achieved.

The amount of preload exerted onto the head by the pressure member can be exactly predefined in a simple manner during assembly by adjusting the externally applied axial force. The preload onto the head generated in this way is reproducible.. The polyaxial bone anchoring device is provided to the surgeon in a pre-assembled manner, in which the pressure member is axially and rotationally fixed by friction in the receiving part to such an extent that it can not fall out or be rotated out of its aligned position. This allows a safe handling by the surgeon. Furthermore, by mounting the pressure member by means of a tool with a predetermined force, a repeatable friction fit, e.g. interference fit connection is achieved.

According to an embodiment of the present invention, the polyaxial bone anchoring device provides for an enlarged pivot angulation of the bone screw by attaching a sleeve-like insert while equally providing high efficiency of fixation. The pivot angle of the bone anchoring element relative to the receiving part maybe equal to or greater than 45° measured from the straight position. This renders the bone anchoring device particularly suitable for the application of lateral mass fixation for example in the cervical spine. The locking mechanism for locking the bone anchoring element and the sleeve-like insert piece provides a high clamping force on a small surface. Therefore, the locking mechanism is efficient.

The bone anchoring device can be designed as a top-loading device, wherein the bone anchoring element is inserted from the top or a bottom loading device, wherein the bone anchoring element is inserted from the bottom.

Further features and advantages of the invention will become apparent from the description of embodiments by means of the accompanying drawings.

In the drawings:
- Fig. 1: shows a perspective exploded view of a polyaxial bone anchoring device with a spinal rod according to a first embodiment.
- Fig. 2: shows a perspective view of the bone anchoring device of Fig. 1 in an assembled state.
- Fig. 3a: shows a cross-sectional front view of the bone anchoring device of Fig. 1 and 2 in the assembled state without the rod and without a fixation screw.
- Fig. 3b: shows an enlarged portion of the cross-sectional front view of the bone anchoring device according to Fig. 3a.
- Fig. 4a: shows a cross-sectional front view of a receiving part according to a first embodiment.
- Fig. 4b: shows a cross-sectional side view of the receiving part according to the first embodiment.
- Fig. 4c: shows a top view of the receiving part according to the first embodiment.
- Fig. 4d: shows a perspective top view of the receiving part according to the first embodiment.
- Fig. 4e: shows a perspective bottom view of the receiving part according to the first embodiment.
- Fig. 5a: shows a cross-sectional front view of a pressure member according to a first embodiment.
- Fig. 5b: shows a side view of the pressure member according to the first embodiment.
- Fig. 5c: shows a cross-sectional top view of the pressure member according to the first embodiment.
- Fig. 5d: shows a perspective bottom view of the pressure member according to the first embodiment.
- Fig. 5e: shows a perspective top view of the pressure member according to the first embodiment.
- Fig. 6a: shows a cross-sectional front view of a sleeve-like insert.
- Fig. 6b: shows a perspective top view of the sleeve-like insert.
- Fig. 6c: shows a top view of the sleeve-like insert.
- Fig. 6d: shows a perspective bottom view of the sleeve-like insert.
- Figs. 7a to 7h: show steps of assembling the sleeve-like insert, the receiving part, the bone anchoring element and the pressure member.
- Fig. 8a: shows a cross-sectional view perpendicular to the rod channel axis of a bone anchoring device in an assembled state without rod and fixation screw according to a second embodiment.
- Fig. 8b: shows an enlarged portion of the cross-sectional view of the bone anchoring device according to Fig. 8a.
- Fig. 9: shows a perspective view of a receiving part according to a second embodiment.
- Fig. 10: shows a perspective view of a pressure member according to a second embodiment.
- Fig. 11a: shows a cross-sectional view perpendicular to the rod channel axis of a bone anchoring device in an assembled state without rod and fixation screw according to a third embodiment.
- Fig. 11b: shows an enlarged portion of the cross-sectional view of the bone anchoring device according to Fig. 10a.
- Fig. 12: shows a perspective view of a receiving part according to a third embodiment.
- Fig. 13: shows a perspective view of a pressure member according to a third embodiment.
- Fig. 14: shows a cross-sectional view perpendicular to the rod channel axis of a bone anchoring device in an assembled state without rod and fixation screw according to a fourth embodiment.
- Fig. 15: shows a cross-sectional view perpendicular to the rod channel axis of a bone anchoring device in an assembled state without rod and fixation screw according to a fifth embodiment.

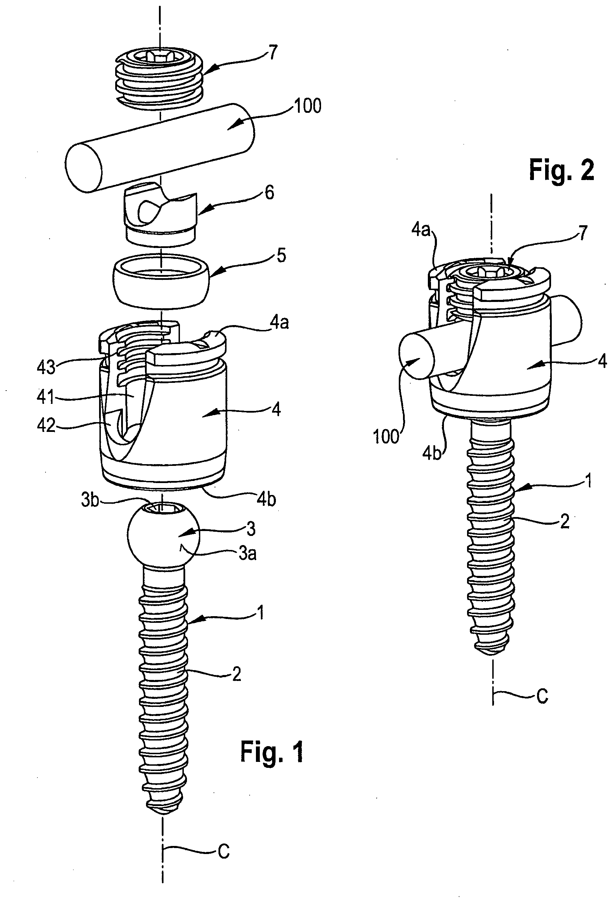

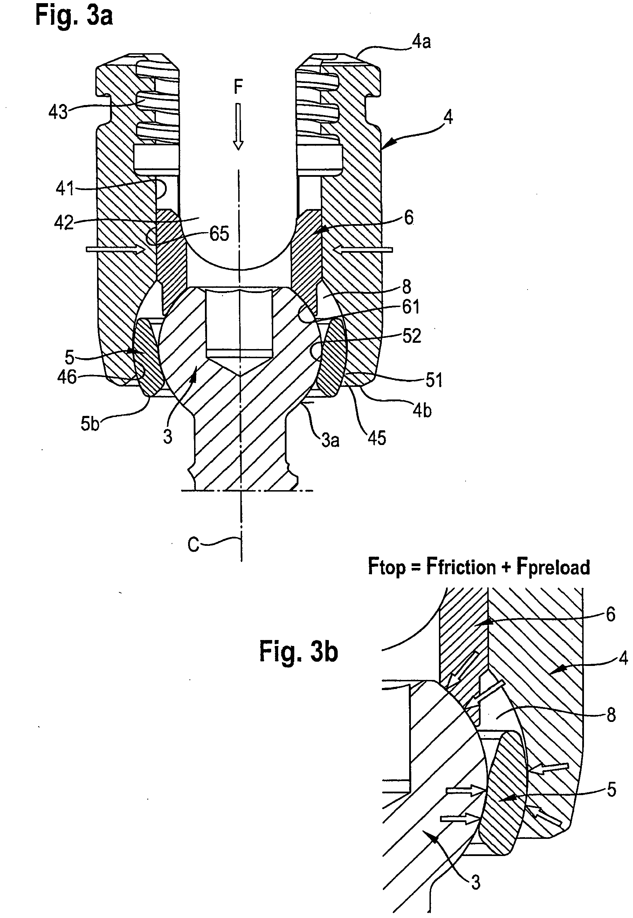

As shown in Figs. 1 to 3b, a polyaxial bone anchoring device according to a first embodiment comprises a bone anchoring element 1 in the form of a bone screw having a threaded shaft 2 and a head 3. The head 3 typically has a spherically-shaped outer surface portion 3a and a recess 3b at its free end for engagement with a tool, e.g. a driver. The head 3 is held in a receiving part 4 that couples the bone anchoring element 1 to a stabilization rod 100. In an assembled state, in the receiving part 4, a sleeve-like insert piece 5 providing a seat for the head 3 and a pressure member 6 for exerting pressure onto the head 3 of the bone anchoring element 1 are arranged. Furthermore, a fixation element in the form of a fixation screw 7 is provided for securing and fixing the rod 100 in the receiving part 4. A bone anchoring device without the sleeve-like insert piece 5 is also possible. In that case, the seat for the head 3 is provided at the receiving part 4 directly.

As can be seen from Figs. 1 to 4c the receiving part 4 has a top end 4a and a bottom end 4b, a central axis C and a coaxial bore 41 extending from the top end 4a in the direction of the bottom end 4b. Adjacent to the top end 4a an U-shaped recess 42 is provided that forms a channel for receiving the rod 100. By means of the U-shaped recess 42, two free legs are formed, which are provided with an internal thread 43 cooperating with the outer thread of the fixation screw 7 in an assembled state (see Fig. 2).

The coaxial bore 41 opens into an accommodation space 8 provided in the lower part of the receiving part 4. The accommodation space 8 has a lower opening 45 at the bottom end 4b of the receiving part 4. The accommodation space 8 further comprises a seat portion 46 near the bottom end 4b of the receiving part 4, in which the sleeve-like insert piece 5 is seated. The seat portion 46 has a spherical shape in order to provide a socket for a ball and socket joint that is formed by the sleeve-insert piece 5 and the receiving part 4. It should be noted that the seat portion 46 can also be tapered or can have another shape that can be used to realize a ball and a socket joint. The inner diameter of the lower opening 45 is smaller than the inner diameter of the accommodation space 8. It shall be noted that the inner diameter of the coaxial bore 41 does not need to be constant between the top end 4a and the accommodation space 8. It may have different portions with different diameters.

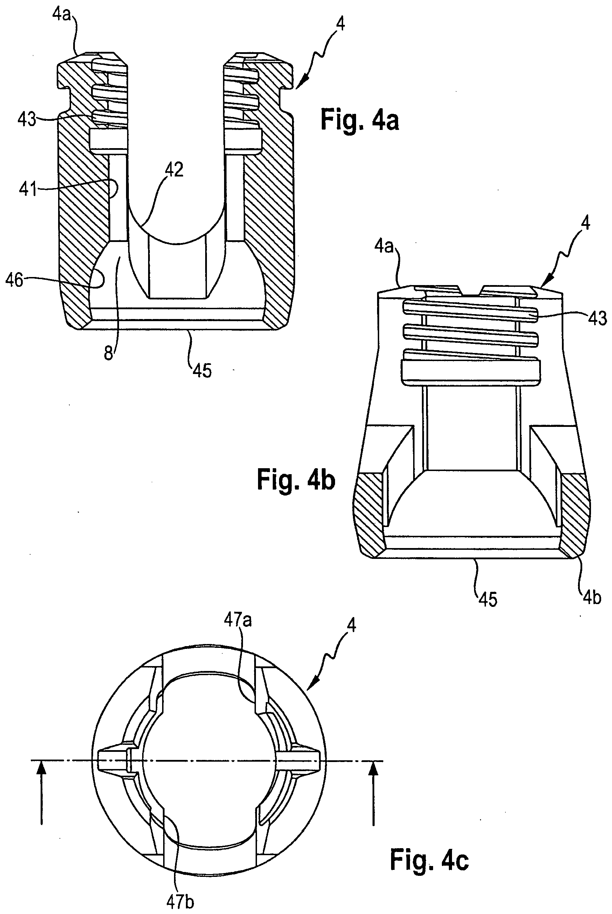

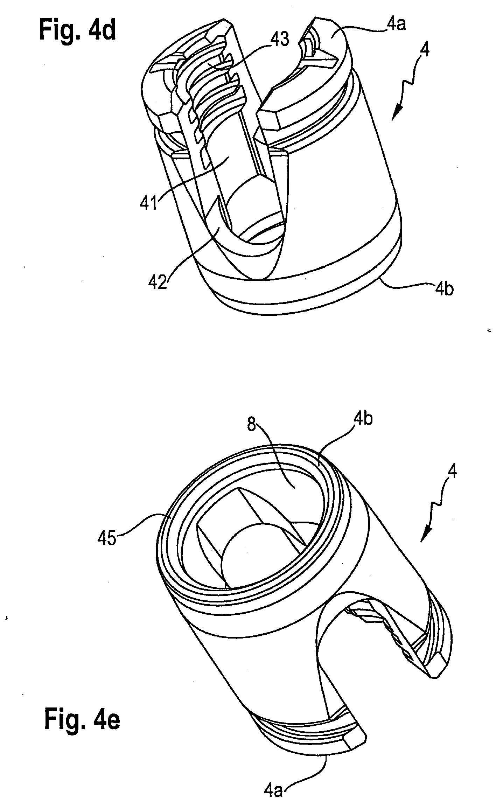

In order to allow the sleeve-like insert piece 5 to be introduced from the top end 4a, two opposed recesses 47a, 47b (see Fig. 4c) are provided in the inner wall of the coaxial bore 41 and the accommodation space 8. The recesses 47a, 47b are aligned with the U-shaped recess 42. They extend from the bottom of the U-shaped recess 42 into the accommodation space 8. The size of the recesses 47a, 47b is such that the sleeve-like insert piece 5 can be introduced from the top end in a 90° tilted position, i.e. the width of the recesses 47a, 47b is greater than the height of the sleeve-like insert piece 5 in its axial direction. The recesses 47a, 47b extend into the accommodation space 8 to such an extent, that tilting of the sleeve-like insert piece 5 into the seat 46 is possible.

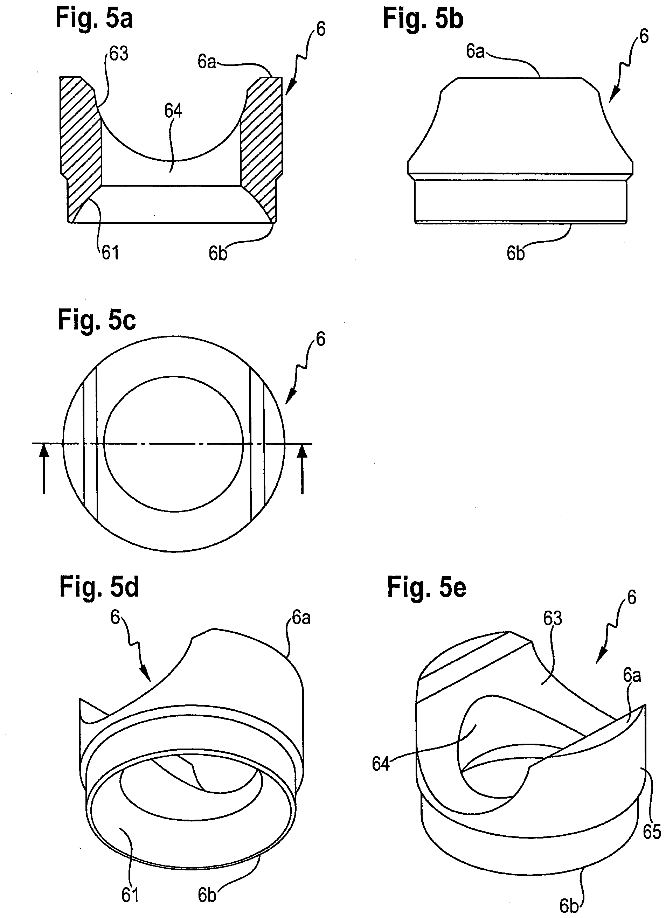

The pressure member 6 is shown in particular in Figs. 5a to 5e. The pressure member 6 is substantially cylindrical with an outer diameter that allows the pressure member 6 to be moved within the coaxial bore 41 and the accommodation space 8, e.g. by means of a tool. However, an outer diameter of the pressure member 6 is slightly larger than an inner diameter of the coaxial bore 41 to achieve an interference fit. or press fit connection between the inner surface of the coaxial bore 41 and the outer surface 65 of the pressure member 6. It is also possible that only parts of the mentioned surfaces form the interference fit. The pressure member 6 has an upper end 6a and a lower edge 6b. Adjacent its lower edge 6b, the pressure member 6 comprises a recess 61 with a spherical shape that matches the shape of the outer spherical surface portion 3a of the head 3. At the upper end 6a, the pressure member 6 comprises a cylindrical recess 63 for receiving the rod 100 therein. Furthermore, the pressure member 6 has a coaxial bore 64 for allowing access to the screw head 3 with a tool in an assembled state. By the coaxial bore 64 and the cylindrical recess 63 two legs are formed facing the top end 4a. The coaxial bore 64 is also configured to allow a portion of the head 3 to extend therethrough, when the bone anchoring element is in a pivoted condition.

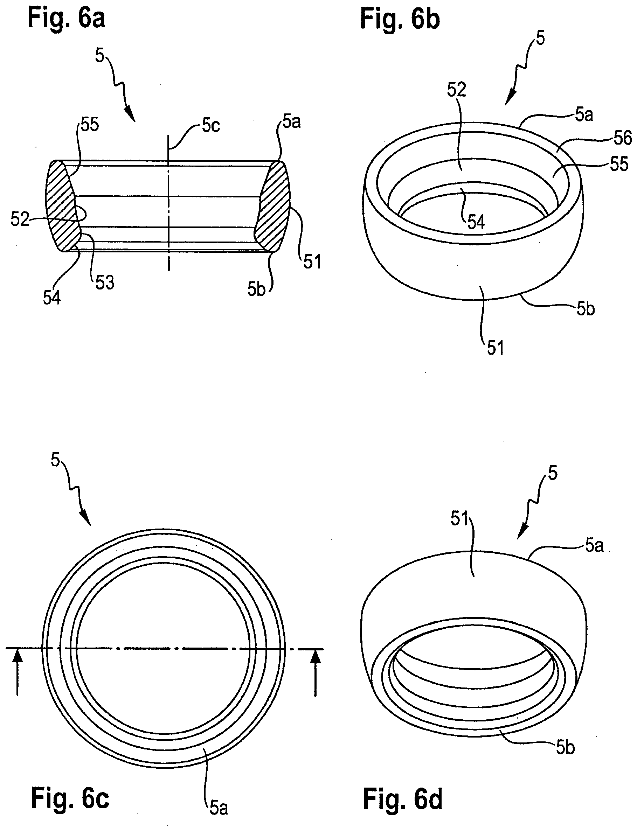

The sleeve-like insert piece 5 is shown in particular in Figs. 6a-6d. The sleeve-like insert piece 5 comprises an upper edge 5a and a lower edge 5b. Between the upper edge 5a and the lower edge 5b, the sleeve-like insert piece 5 comprises a spherical-shaped outer surface portion 51. The largest outer diameter of the sleeve-like insert piece 5 is greater than the inner diameter of the lower opening 45 of the receiving part 4. Hence, the sleeve-like insert piece 5 can not escape through the lower opening 45, when it is seated in the receiving part 4. The dimension of the outer spherical surface portion 51 corresponds to the spherical-shaped seat portion 46 of the receiving part 4 in such a way that the sleeve-like insert piece 5 can pivot and rotate in the receiving part 4, when it is seated in the seat portion 46. When the sleeve-like insert piece 5 rests in the seat portion 46 such that its centre axis 5c is coaxial with the centre axis C of the receiving part 4, the lower edge 5b projects out of the lower opening 45. When the sleeve-like insert piece 5 is pivoted in the receiving part 4, at least a portion of the lower edge 5b still projects out of the lower opening 45.

The sleeve-like insert piece 5 is hollow and comprises a central inner portion 52 that is spherically-shaped with a radius corresponding to the radius of the spherically-shaped outer surface portion 3a of the head 3 of the bone anchoring element 1. The lower end of the central portion 52 forms a shoulder 53. The inner diameter of the shoulder 53 is smaller than the largest outer diameter of the spherical head 3 so that the head 3 can rotate and pivot in the central spherical portion 52 of the sleeve-like insert piece 5 similar to a ball and socket joint. Between the shoulder 53 and the lower edge 5b a tapered portion 54 is provided that tapers outwards to allow angulation of the bone anchoring element 1 until the shaft 2 comes into contact with the lower edge 5b. Between the spherical central portion 52 and the upper edge 5a a tapered portion 55 is provided, which tapers outwards. The inner diameter of the tapered portion 55 and of the transition between the tapered portion 55 and the spherical central portion 52 is always greater than the largest outer diameter of the head 3, so that the head 3 can be inserted from the upper edge 5a into the sleeve-like insert piece 5. At the upper edge 5a, a chamfered portion 56 is provided that may serve as a stop for the pressure member 6.

The center points of the spherical central portion 52 and the outer spherical portion 51 may be offset in such a way that the centre point of the inner central spherical portion 52 is shifted in the direction towards the bottom end 4b. By means of this, the range of angulation for the bone anchoring element 1 can be further increased. The height of the sleeve-like insert piece 5 in axial direction is less than the height of the head 3 in axial direction such that, when the head 3 is inserted into the sleeve-like insert piece 5, still a portion of the spherical outer surface 3a of the head 3 projects from the upper edge 5a of the sleeve-like insert piece 5 as can be seen from Fig. 3a.

The sleeve-like insert piece 5 and the anchoring element 1 are independently pivotable when the shaft 2 of the anchoring element 1 and the lower edge 5b of the sleeve-like insert piece 5 are out of contact. When the shaft 2 of the bone anchoring element 1 is pivoted and engages the lower edge 5b of the sleeve-like insert piece 5, further pivoting of the bone anchoring element 1 causes the sleeve-like insert piece 5 to pivot together with the bone anchoring element 1. When the pressure member 6 is in contact with the head 3, there is a gap between the pressure member 6 and the sleeve-like insert piece 5.

As indicated by arrows in Figs. 3a, 3b a force F which is provided from above in the figures divides in frictional resistance and preload force (indicated by the small arrows in Fig. 3b) which holds the head with respect to the receiving part 4 in a desired angular orientation by friction. The frictional resistance results from the interference fit connection between the pressure member 6 and the receiving part 4, wherein the outer diameter of at least one portion of the pressure member 6 is slightly larger than the inner diameter of the corresponding portion of the receiving part 4. The preload force acting on the head 4 leads to a small elastic preload of the whole system.

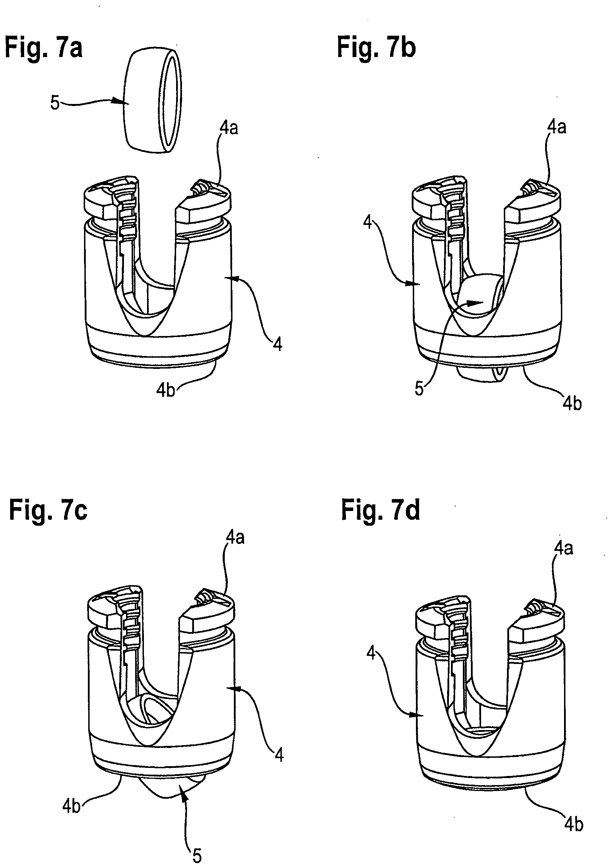

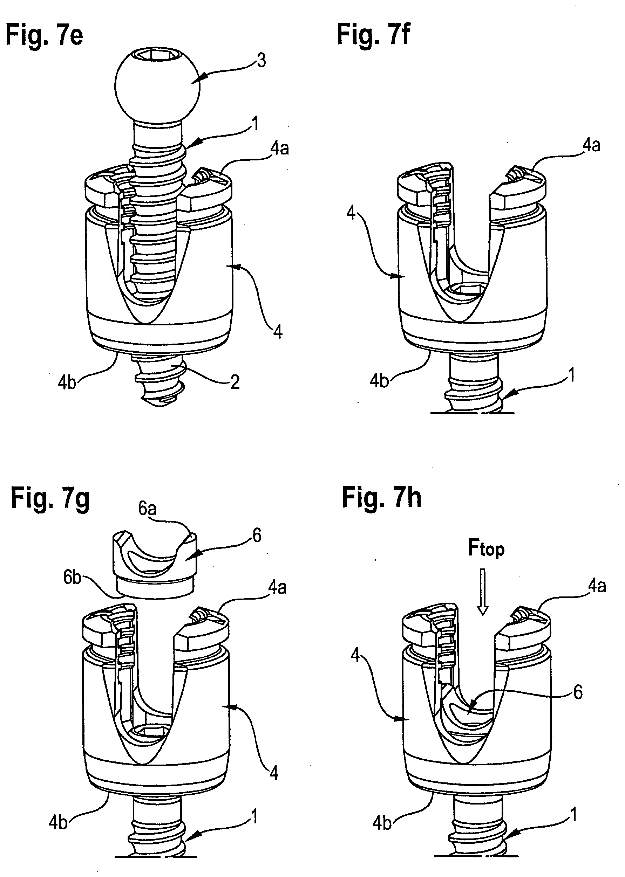

The steps of pre-assembling the bone anchoring device according to the first embodiment are shown with respect to Figs. 7 a) to 7 h). The bone anchoring device according to the first embodiment may be pre-assembled in such a way that first the sleeve-like insert piece 5 is tilted by 90° and inserted into the receiving part 4 at the position of the U-shaped recess 42 as can be seen from Figs. 7a and 7b. As shown in Fig. 7b the sleeve-like insert piece 5 is moved downwards into the accommodation space 8. Since the outer diameter of the sleeve-like insert piece 5 is larger than the inner diameter the lower opening 45 of the receiving part 4, the sleeve-like insert piece 5 can not escape through the lower edge of the lower opening 45. Then, as shown in Figs. 7c and 7d the sleeve-like insert piece 5 is tilted so that it finally is seated in the seat portion 46, as shown in Fig. 7d.

Thereafter, the bone anchoring element 1 is inserted from the top end 4a of the receiving part 4 until the outer surface portion 3a of the head 3 engages the seat portion 52 of the sleeve-like insert piece 5 as can be seen from Figs. 7e and 7f. Then, the pressure member 6 is inserted from the top end 4a as can be seen from Fig. 7g by applying a predefined force from the top as indicated by the arrow in Fig. 7h. The pressure member 6 is arranged in an aligned position, in which the cylindrical recess 63 is aligned with the U-shaped recess 42 of the receiving part 4 for receiving the rod 100. Dependent on the degree of the interference fit connection it may be necessary to use a tool for pushing down the pressure member 6 into the receiving part 4. The predefined force from above may be generated manually or by a tool, for example and may be constant and/or force- or path-controlled.

The bone anchoring device as a whole or in parts is made of a bio-compatible material, such as a bio-compatible metal, for example titanium, stainless steel, bio-compatible alloy, such as nitinol, or of bio-compatible plastic material, such as, for example, polyetheretherketone (PEEK).

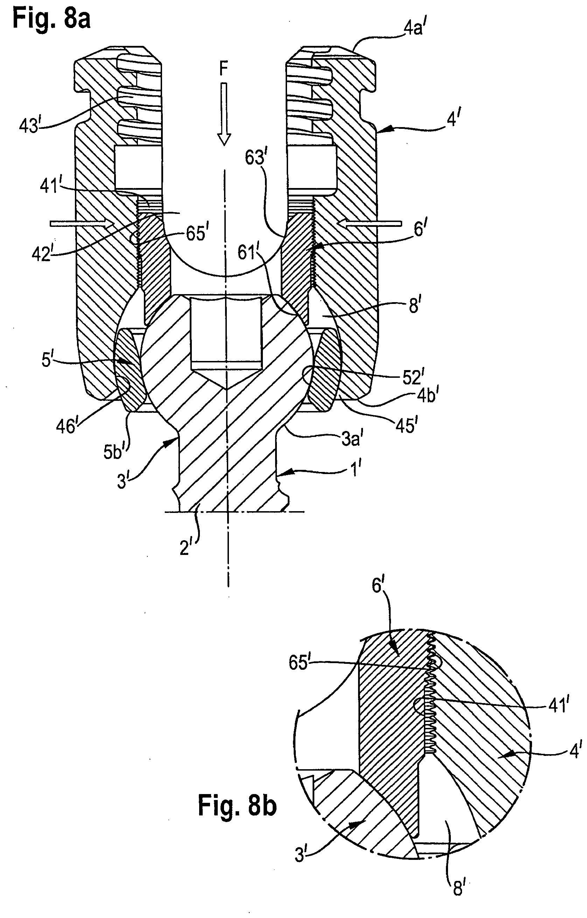

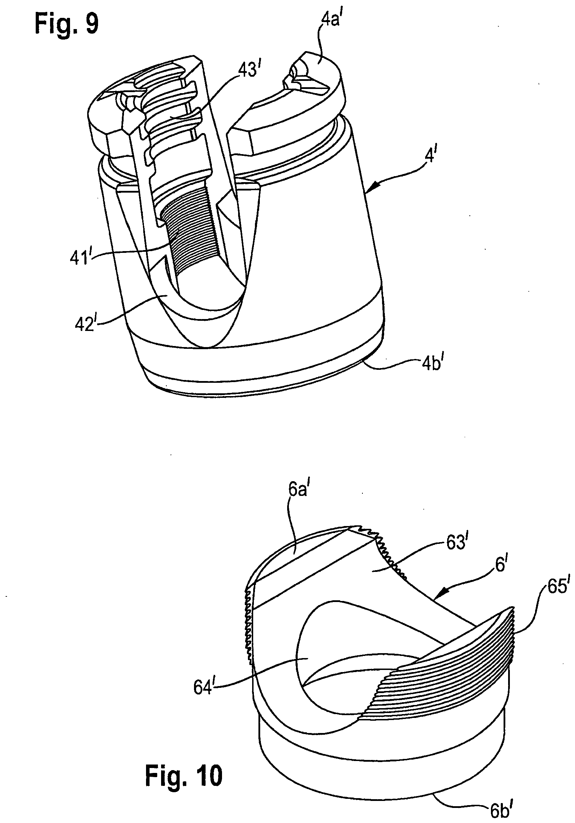

Figs. 8a to 10 show a second embodiment of the bone anchoring device. Parts and portions, which are the same or similar to those of the first embodiment, are designated with the same reference numerals and the description thereof is not repeated. The bone anchoring device according to the second embodiment differs from the bone anchoring device of the first embodiment by the construction of the outer surface 65' of the pressure member 6' and the corresponding surface of the coaxial bore 41' of the receiving part 4'. All other parts are identical to those of the first embodiment.

As can be seen especially from Fig. 8b, the surface of the coaxial bore 41' is structured, for example roughened or fluted or grooved or ridged. The outer surface of the pressure member 6' is also structured, such as roughened or fluted or grooved or ridged. The surface interaction of the two structured surfaces prevents the pressure member 6' from moving backwards towards the first end 4a'. Therefore, the holding function of the interference fit connection is further increased. It is also possible that only one of the surfaces is structured.

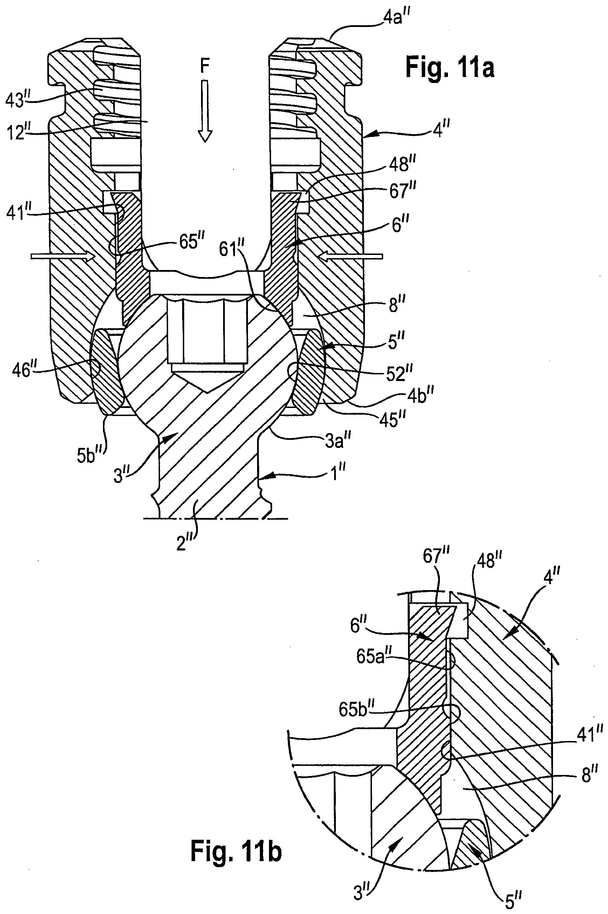

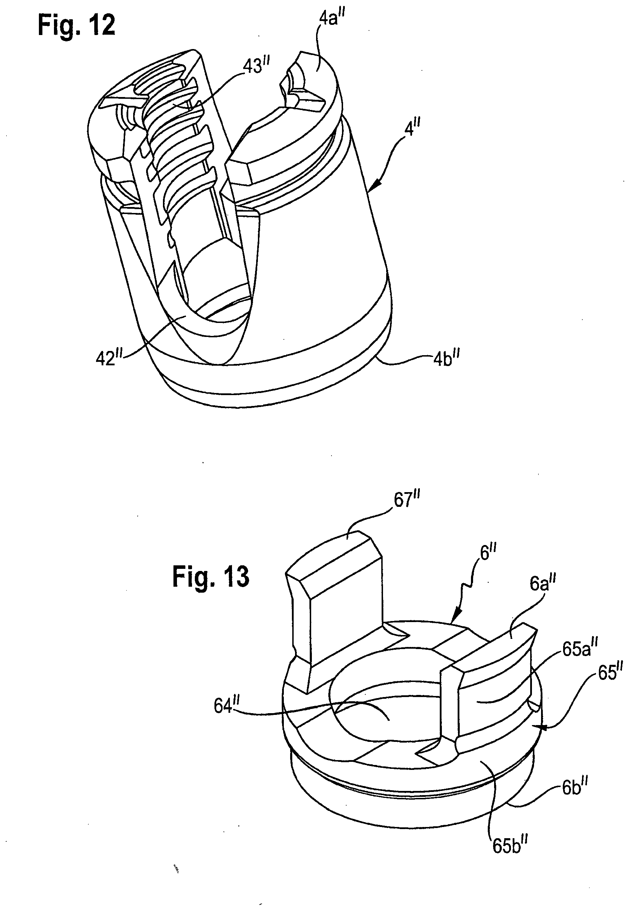

Figs. 11a to 13 show a third embodiment of the bone anchoring device. Parts and portions, which are the same or similar to those of the first embodiment, are designated with same reference numerals and the description thereof is not repeated. The bone anchoring device according to the third embodiment differs from the bone anchoring device of the first embodiment by the construction of the pressure member 6" and the corresponding portions of the receiving part 4". All other parts are identical to those of the first embodiment.

Referring to the outer surface 65" of the pressure member 6" having an upper portion 65a" and a lower portion 65b" with slightly different outer diameters, an interference fit is in this case only present at the lower portion 65b" of the outer surface of the pressure member 6", which contacts the surface of the coaxial bore 41" of the receiving part 4". That means, only the diameter of the lower portion 65b" is slightly larger than the diameter of the coaxial bore 41". The diameter of the upper portion 65a" may be the same or smaller than the diameter of the coaxial bore 41". The upper portion 65a" further comprises two projections 67" at the free ends of the legs of the pressure member 6", wherein the projections 67" extend radially outwards and which latch into an annular groove 48", which is provided in the coaxial bore 41" of the receiving part 4", when the pressure member 6" is inserted into the receiving part 4". Due to the larger outer diameter of the projections 67" with respect to the diameter of the coaxial bore 41" the legs of the pressure member 6" are compressed towards each other and elastically expand when the projections 67" snap into the groove 48".

The pre-assembling of the bone anchoring device according to the second and third embodiment corresponds to the pre-assembling according to the first embodiment.

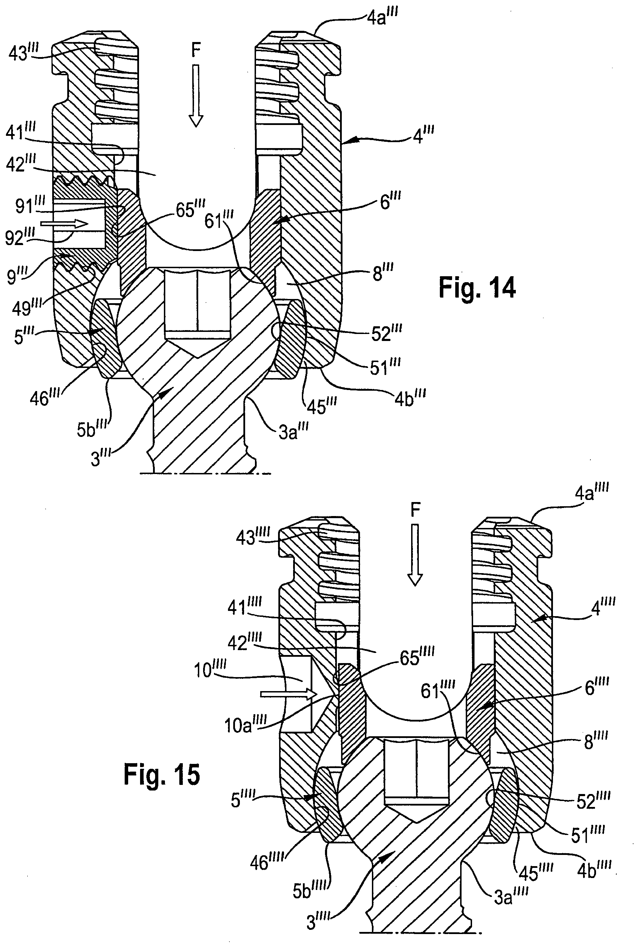

Fig. 14 shows a fourth embodiment of the bone anchoring device, which is not according to the invention. Parts and portions, which are the same or similar to those of the first embodiment, are designated with same reference numerals and the description thereof is not repeated. The bone anchoring device according to the fourth embodiment differs from the bone anchoring device according to the first embodiment in that there is no interference fit connection between the receiving part 4"' and the pressure member 6"', i.e. the outer diameter of at least one portion of the pressure member 6"' is equal to or smaller than the inner diameter of the corresponding portion of the receiving part 4"'. Instead, a set screw 9"' is provided which is screwed into a through bore 49"' which is located in one leg of the receiving part 4"' during assembly for fixing the pressure member 6"' relative to the receiving part 4"'. The set screw 9"' comprises an engagement portion 92'" for engagement with a tool and a flat bottom side 91"' cooperating with the outer surface 65"' of the pressure member 6"'. All other parts are identical to those of the first embodiment.

The first steps of pre-assembling the bone anchoring device according to the fourth embodiment correspond to the pre-assembling according to the first embodiment. A predefined force is applied on the pressure member 6"' from above to define the preload force acting on the head 3"'. After that the pressure member 6"' is frictionally fixed by screwing in the set screw 9"'. The set screw 9"' only acts radially, i.e. perpendicular to the axis C"', onto the outer surface 65"' of the pressure member 6"' by which the pressure member 6"" is held in place. Therefore, the preload force acing on the head 3"" is maintained.

Fig. 15 shows a fifth embodiment of the bone anchoring device, which is also not according to the invention. Parts and portions, which are the same or similar to those of the first embodiment, are designated with same reference numerals and the description thereof is not repeated. The bone anchoring device according to the fifth embodiment differs from the bone anchoring device according to the first embodiment in that there is no interference fit connection between the receiving part 4"" and the pressure member 6"", i.e. the outer diameter of at least one portion of the pressure member 6"" is equal to or smaller than the inner diameter of the corresponding portion of the receiving part 4"". A crimping blind hole 10"" is provided which is located in one leg of the receiving part 4"". All other parts are identical to those of the first embodiment.

The first steps of pre-assembling the bone anchoring device according to the fifth embodiment correspond to the pre-assembling according to the first embodiment. A predefined force is applied on the pressure member 6"" from above to define the preload force acting on the head 3"". After that, the pressure member 6"" is frictionally fixed by crimping by means of a crimping tool. Hereby a deformable portion 10a"" of the receiving part 4"" adjacent to the crimping blind hole 10"" is deformed and deformed material radially exerts a pressure force onto the outer surface 65"" of the pressure member 6"" by which the pressure member 6"" is held in place. Therefore, the preload force acting on the head 3"" is maintained.

Further modifications of the embodiments described are conceivable. For example, for the bone anchoring element, all kinds of anchoring elements can be used and combined with the receiving part. These bone anchoring elements are e.g. screws of different lengths, with different diameters, cannulated screws, screws with different thread forms, nails, hooks, etc. The head and the shaft may also be separate parts, that are connectable to each other.

Modifications of the receiving part include a recess for the rod, which is inclined or open to the side, instead of the U-shaped recess, which is perpendicular to the central axis. Other kinds of locking devices including outer nuts, outer caps, bayonet locking devices or others are also possible. In all embodiments, the inner surface portion of the pressure member that contacts the head, need not necessarily to be spherical-shaped. It can have another shape that is suitable to exert pressure onto the head.

It is also possible to use a two-part locking device for separately fixing the rod and the head of the bone anchoring element.

It is also possible that the pressure member is prevented from rotation by additional crimping.

It may be noted that according to specific embodiments of the polyaxial bone anchoring device, the pressure member 6" may comprise two projections 67" extending radially outwards which are formed such that the projections 67" latch into an annular groove 48" which is provided in the bore 41", when the pressure member 6" is inserted into the receiving part 4".

According to other specific embodiments of the polyaxial bone anchoring device, which may be combined with the preceding embodiment, the pressure member 6, 6', 6", 6"', 6"" has an upper end 6a, 6a', 6a", 6a"', 6a"" and a lower edge 6b, 6b', 6b", 6b"', 6b"", 6"', 6"" and wherein adjacent to the lower edge 6b, 6b', 6b", 6b"', 6b"", the pressure member 6, 6', 6", 6"', 6"" comprises a recess 61, 61', 61", 61"', 61"" with a spherical shape that matches the shape of an outer spherical surface portion 3a, 3a', 3a", 3a"', 3a"" of the head 3, 3', 3", 3"', 3"".

According to other specific embodiments of the polyaxial bone anchoring device, which may be combined with one or both the preceding embodiments, a sleeve-like insert piece 5, 5', 5", 5"', 5"" is provided encompassing a portion of the head 3, 3', 3", 3"', 3"" having an outer spherical surface portion 51, 51', 51", 51"', 51"" and being configured to pivot in the receiving part 4, 4', 4", 4"', 4"" and wherein a lower edge 5b, 5b', 5b", 5b"', 5b"" of the sleeve-like insert piece 5, 5', 5", 5"', 5"" extends through the lower opening 45, 45', 45", 45"', 45"", when the sleeve-like insert piece 5, 5', 5", 5"', 5"" is seated in the receiving part 4, 4', 4", 4"', 4"" in a position, in which its sleeve axis 5c, 5c', 5c", 5c"', 5c"" is coaxial with the longitudinal axis C.

It may further be noted that in embodiments of the method according to the invention, the radially acting force results from an interference fit connection between the pressure member 6, 6', 6" and the receiving part 4, 4', 4".

According to another specific embodiments of the method, that may also be combined with the preceding embodiment, the radially acting force results from screwing in a set screw 9"' into a through bore 49"' in the receiving part 4"' exerting pressure onto the pressure member 6"'.

According to still another specific embodiments of the method, that may be combined with one or both of the preceding embodiments, the radially acting force results from crimping, wherein deformable material of the receiving part 4"" exerts pressure onto the pressure member 6".

## Claims

1. A polyaxial bone anchoring device comprising
a bone anchoring element (1, 1', 1") having a head (3, 3', 3") and a shaft (2, 2', 2") for anchoring in the bone,
a receiving part (4, 4', 4") for coupling the bone anchoring element (1, 1', 1") to a rod (100), the receiving part (4, 4', 4") comprising an accommodation space (8, 8', 8") for accommodating the head (3, 3', 3") and a coaxial bore (41, 41', 41") being in communication with the accommodation space (8, 8', 8"), the coaxial bore (41, 41', 41") having a bore axis,
a pressure member (6, 6', 6") configured to move in the bore (41, 41', 41") and comprising a first surface for engaging the head (3, 3', 3") and a second surface on which the rod (100) acts, wherein
the pressure member (6, 6', 6") is configured to assume a first position in which it exerts a preload onto the head (3, 3', 3") that results from friction between the first surface and the head (3, 3', 3") to enable the shaft (2, 2', 2") to be maintained in a desired angular position before locking the head (3, 3', 3") in the receiving part (4, 4', 4"), and
a second position in which the head (3, 3', 3") is locked with respect to the receiving part (4, 4', 4"), and wherein
the first position is obtained by moving the pressure member (6, 6', 6") by the action of a predefined force acting onto the pressure member (6, 6', 6") in an axial direction and wherein
the pressure member (6, 6', 6") is maintained in the first position by interaction with the receiving part (4, 4', 4") and can be released from the first position through action of an axial force,
**characterized in that** the pressure member (6, 6', 6") is a single piece substantially cylindrical member and an outer diameter of the pressure member (6, 6', 6") is slightly larger than an inner diameter of the coaxial bore (41, 41', 41") to achieve an interference fit connection between an inner surface of the coaxial bore and the outer cylindrical surface (65, 65', 65b") of the pressure member, or between parts of the inner surface of the coaxial bore and of the outer cylindrical surface of the pressure member.

2. The polyaxial bone anchoring device of claim 1, wherein the pressure element is maintained in the first position by action of a radial force that generates friction between the pressure member (6, 6', 6") and at least a portion of the receiving part (4, 4', 4").

3. The bone anchoring device of one of the claims 1 to 2, wherein the surface of the bore (41') is structured in at least a portion thereof.

4. The bone anchoring device of claim 3, wherein the surface of the bore (41') is roughened, fluted, grooved or ridged in at least a portion thereof.

5. The bone anchoring device of one of the claims 1 to 4, wherein the outer surface (65') of the pressure member (6') is structured in at least a portion thereof.

6. The bone anchoring device of claim 5, wherein the outer surface (65') of the pressure member (6') is roughened, fluted, grooved or ridged in at least a portion thereof.

7. The bone anchoring device of one of claims 3 to 6, wherein the structured surface of the bore (41') and the structured outer surface (65') of the pressure member (6') is respectively structured such as to prevent the pressure member (6') from moving backwards towards a first top end (4a') of the receiving part (4').

8. The bone anchoring device of one of the claims 3 to 7, wherein the structured portion of the outer surface (65') extends partially circumferentially around a portion of the pressure member.

9. A method for manufacturing a bone anchoring device according to one of the claims 1 to 8, including the steps:
arranging the head (3, 3', 3") of the bone anchoring element (1, 1', 1") in the receiving part (4, 4', 4"),
arranging the pressure member (6, 6', 6") in the receiving part (4, 4', 4")
applying a predetermined axial force onto the pressure member (6, 6', 6") to create a preload force acting on the head (3, 3', 3") of the bone anchoring element (1, 1', 1"), wherein the preload force is maintained by a radially acting force.

## Patentansprüche

1. Polyaxiale Knochenverankerungsvorrichtung, aufweisend
ein Knochenverankerungselement (1, 1', 1"), das einen Kopf (3, 3', 3") und, zum Verankern in dem Knochen, einen Schaft (2, 2', 2") hat,
ein Aufnahmeteil (4, 4', 4") zum Koppeln des Knochenverankerungselements (1, 1', 1") an einen Stab (100), wobei das Aufnahmeteil (4, 4', 4") einen Aufnahmeraum (8, 8', 8") zum Aufnehmen des Kopfs (3, 3', 3") und eine koaxiale Bohrung (41, 41', 41"), die mit dem Aufnahmeraum (8, 8', 8") verbunden ist, aufweist, wobei die koaxiale Bohrung (41, 41', 41") eine Bohrungsachse hat,
ein Druckelement (6, 6', 6"), das konfiguriert ist, sich in der Bohrung (41, 41', 41") zu bewegen, und eine erste Oberfläche zum Eingriff des Kopfs (3, 3', 3") und eine zweite Oberfläche aufweist, auf die der Stab (100) wirkt, wobei
das Druckelement (6, 6', 6") konfiguriert ist, eine erste Position, in der es eine Vorspannung auf den Kopf (3, 3', 3"), die aus einer Reibung zwischen der ersten Oberfläche und dem Kopf (3, 3', 3") resultiert, ausübt, um es dem Schaft (2, 2', 2") zu ermöglichen, in einer gewünschten Winkelposition beibehalten zu werden bevor der Kopf (3, 3', 3") in dem Aufnahmeteil (4, 4', 4") festgestellt wird, und
eine zweite Position einzunehmen, in der der Kopf (3, 3', 3") bezüglich des Aufnahmeteils (4, 4', 4") festgestellt wird, und wobei
die erste Position durch Bewegen des Druckelements (6, 6', 6") durch die Wirkung einer vordefinierten Kraft, die in einer axialen Richtung auf das Druckelement (6, 6', 6") wirkt, erreicht wird, und wobei
das Druckelement (6, 6', 6") durch ein Zusammenwirken mit dem Aufnahmeteil (4, 4', 4") in der ersten Position beibehalten wird, und durch eine Wirkung einer axialen Kraft von der ersten Position freigegeben werden kann, **dadurch gekennzeichnet, dass**
das Druckelement (6, 6', 6") ein einstückiges, im wesentlichen zylindrisches Element ist und ein Außendurchmesser des Druckelements (6, 6', 6") geringfügig größer ist als ein Innendurchmesser der koaxialen Bohrung (41, 41', 41"), um eine kraftschlüssige Verbindung zwischen einer inneren Oberfläche der koaxialen Bohrung und der äußeren zylindrische Oberfläche (65, 65', 65') des Druckelements, oder zwischen Teilen der inneren Oberfläche der koaxialen Bohrung und der äußeren zylindrischen Oberfläche des Druckelements, zu erreichen.

2. Die polyaxiale Knochenverankerungseinrichtung gemäß Anspruch 1, wobei das Druckelement durch eine Wirkung einer radialen Kraft, die eine Reibung zwischen dem Druckelement (6, 6', 6") und zumindest einem Abschnitt des Aufnahmeteils (4, 4', 4") erzeugt, in der ersten Position beibehalten wird.

3. Die Knochenverankerungseinrichtung gemäß Anspruch 1 oder 2, wobei die Oberfläche der Bohrung (41') zumindest in einem Abschnitt davon strukturiert ist.

4. Die Knochenverankerungseinrichtung gemäß Anspruch 3, wobei die Oberfläche der Bohrung (41') zumindest in einem Abschnitt aufgeraut, geriffelt, gerillt oder gezahnt, ist.

5. Die Knochenverankerungseinrichtung gemäß einem der Ansprüche 1 bis 4, wobei die äußere Oberfläche (65') des Druckelements (6') zumindest in einem Abschnitt davon strukturiert ist.

6. Die Knochenverankerungseinrichtung gemäß Anspruch 5, wobei die äußere Oberfläche (65') des Druckelements (6') zumindest in einem Abschnitt aufgeraut, geriffelt, gerillt oder gezahnt ist.

7. Die Knochenverankerungseinrichtung gemäß einem der Ansprüche 3 bis 6, wobei die strukturierte Oberfläche der Bohrung (41') und die strukturierte äußere Oberfläche (65') des Druckelements (6') entsprechend so strukturiert sind, dass das Druckelement (6') daran gehindert wird, rückwärts in Richtung auf das obere Ende (4a') des Aufnahmeteils (4') bewegt zu werden.

8. Die Knochenverankerungseinrichtung gemäß einem der Ansprüche 3 bis 7, wobei sich der strukturierte Abschnitt der äußeren Oberfläche (65') teilumfänglich um einen Abschnitt der Druckelements (6') herum erstreckt.

9. Verfahren zum Herstellen einer Knochenverankerungsvorrichtung gemäß einem der Ansprüche 1 bis 8, die Schritte enthaltend:
Anordnen des Kopfs (3, 3', 3") des Knochenverankerungselements (1, 1', 1") in dem Aufnahmeteil (4, 4', 4"),
Anordnen des Druckelements (6, 6', 6") in dem Aufnahmeteil (4, 4', 4"),
Anlegen einer vorbestimmten axialen Kraft auf das Druckelement (6, 6', 6"), um eine Vorspannkraft, die auf den Kopf (3, 3', 3") des Knochenverankerungselements (1, 1', 1") wirkt, zu erzeugen, wobei die Vorspannkraft durch eine radial wirkende Kraft aufrechterhalten wird.

## Revendications

1. Dispositif d'ancrage osseux polyaxial comprenant
un élément d'ancrage osseux (1, 1', 1") ayant une tête (3, 3', 3") et un arbre (2, 2', 2") pour l'ancrage dans l'os,
une partie de réception (4, 4', 4") pour coupler l'élément d'ancrage osseux (1, 1', 1") à une tige (100), la partie de réception (4, 4', 4") comprenant un espace de logement (8, 8', 8") pour loger la tête (3, 3', 3") et un alésage coaxial (41, 41', 41") étant en communication avec l'espace de logement (8, 8', 8"), l'alésage (41, 41', 41") ayant un axe d'alésage,
un élément de pression (6, 6', 6") configuré pour se déplacer dans l'alésage (41, 41', 41") et comprenant une première surface pour se mettre en prise avec la tête (3, 3', 3") et une deuxième surface sur laquelle la tige (100) agit, dans lequel
l'élément de pression (6, 6', 6") est configuré pour adopter une première position dans laquelle il exerce une précharge sur la tête (3, 3', 3") qui résulte de la friction entre la première surface et la tête (3, 3', 3") pour permettre à l'arbre (2, 2', 2") d'être maintenu dans une position angulaire souhaitée avant le verrouillage de la tête (3, 3', 3") dans la partie de réception (4, 4', 4"), et une deuxième position dans laquelle la tête (3, 3', 3") est verrouillée par rapport à la partie de réception (4, 4', 4"), et dans lequel
la première position est obtenue en déplaçant l'élément de pression (6, 6', 6") par l'action d'une force prédéfinie agissant sur l'élément de pression (6, 6', 6") dans une direction axiale et dans lequel
l'élément de pression (6, 6', 6") est maintenu dans la première position par interaction avec la partie de réception (4, 4', 4") et peut être libéré de la première position par l'action d'une force axiale,
**caractérise en ce que** l'élément de pression (6, 6', 6") est un élément monobloc sensiblement cylindrique et un diamètre extérieur de l'élément de pression (6, 6', 6") est légèrement plus grand qu'un diamètre intérieur de l'alésage coaxial (41, 41', 41") pour réaliser une connexion par friction entre une surface intérieure de l'alésage coaxial et une surface cylindrique extérieure (65, 65', 65b") de l'élément de pression, ou entre des parties de la surface intérieure de l'alésage coaxial et de la surface cylindrique extérieure de l'élément de pression.

2. Dispositif d'ancrage osseux polyaxial selon la revendication 1, dans lequel l'élément de pression est maintenu dans la première position par l'action d'une force radiale qui génère une friction entre l'élément de pression (6, 6', 6") et au moins une partie de la partie de réception (4, 4', 4").

3. Dispositif d'ancrage osseux selon la revendication 1 ou 2, dans lequel la surface de l'alésage (41, 41', 41") est structurée dans au moins une partie de celle-ci.

4. Dispositif d'ancrage osseux selon la revendication 3, dans lequel la surface de l'alésage (41') est rendue rugueuse, cannelée, rainurée ou striée dans au moins une partie de celle-ci.

5. Dispositif d'ancrage osseux selon une des revendications 1 à 4, dans lequel la surface extérieure (65') de l'élément de pression (6') est structurée, dans au moins une partie de celle-ci.

6. Dispositif d'ancrage osseux selon la revendication 5, dans lequel la surface extérieure (65') de l'élément de pression (6') est rendue rugueuse, cannelée, rainurée ou striée dans au moins une partie de celle-ci.

7. Dispositif d'ancrage osseux selon une des revendications 3 à 6, dans lequel la surface structurée de l'alésage (41') et la surface extérieure structurée (65') de l'élément de pression (6') est respectivement structurée de manière à empêcher l'élément de pression (6') d'être déplacé en arrière vers l'extrémité supérieure (4a') de la partie de réception (4').

8. Dispositif d'ancrage osseux selon une des revendications 3 à 7, dans lequel la partie structurée de la surface extérieure (65') s'étend partiellement autour d'une partie de l'élément de pression.

9. Procédé de fabrication d'un dispositif d'ancrage osseux selon l'une des revendications 1 à 8, comportant les étapes qui consistent:
à agencer la tête (3, 3', 3") de l'élément d'ancrage osseux (1, 1', 1") dans la partie de réception (4, 4', 4"),
à agencer l'élément de pression (6, 6', 6") dans la partie de réception (4, 4', 4"),
à appliquer une force axiale prédéterminée sur l'élément de pression (6, 6', 6") pour créer une force de précharge agissant sur la tête (3, 3', 3") de l'élément d'ancrage osseux (1, 1', 1", 1"', 1""), dans lequel
la force de précharge est maintenue par une force agissant radialement.
